# EUROPÄISCHE PATENTSCHRIFT

(11) **EP 1 315 462 B1**
(45) Veröffentlichungstag und Bekanntmachung des Hinweises auf die Patenterteilung: **15.07.2009**
(21) Anmeldenummer: 00954257.2
(22) Anmeldetag: 07.09.2000
(51) Int. Cl.: A61B 17/72, F16B 19/10

(54) **VORRICHTUNG ZUR FIXIERUNG VON CHIRURGISCHEN IMPLANTATEN**
DEVICE FOR FIXING SURGICAL IMPLANTS
DISPOSITIF DE FIXATION D'IMPLANTS CHIRURGICAUX

(43) Veröffentlichungstag der Anmeldung: 04.06.2003
(73) Patentinhaber: Synthes GmbH, 4436 Oberdorf (CH)
(72) Erfinder: STEIGER, Peter, CH-3360 Herzogenbuchsee (CH); FRIGG, Robert, CH-2544 Bettlach (CH); ESCHBACH, Lukas, CH-3294 Büren an der Aare (CH)
(74) Vertreter: Lusuardi, Werther
(86) Internationale Anmeldenummer: PCT/CH2000/000478
(87) Internationale Veröffentlichungsnummer: WO 2002/019931

(56) Entgegenhaltungen:
- EP-A- 0 998 878
- WO-A-99/62417
- FR-A- 2 141 020
- FR-A- 2 552 830
- GB-A- 2 054 082
- US-A- 3 065 661
- US-A- 4 810 141

## Beschreibung

Die Erfindung bezieht sich auf eine Vorrichtung zur Frakturbehandlung an Knochen und/oder zur Fixierung von chirurgischen Implantaten, chirurgischen Fäden oder Geweben im oder am Knochen gemäss dem Oberbegriff des Patentanspruchs 1, wie sie beispielsweise aus der EP-A-0998878 als bekannt hervorgeht.

Zur chirurgischen Frakturbehandlung an Knochen oder zur Fixierung von chirurgischen Implantaten, Fäden oder Geweben im oder am Knochen können auch Nieten, insbesondere Blindnieten eingesetzt werden. Besonders geeignet sind Nieten, deren Schliessköpfe aus separaten, relativ weit aufspreizbaren Verankerungszungen gebildet werden. Aus dem nicht-medizinischen Bereich, beispielsweise aus der GB 2 054 082 TUCKER FASTENERS sind bereits solche Blindnieten bekannt. Die Verankerungszungen werden durch axiales Zerreissen der Wand des Nietenschaftes am vorderen Ende der Blindniete mittels eines pyramidenförmigen, scharfkantigen Schliesskopfes, welcher in den hohlzylindrischen Nietenschaft vom vorderen Ende her eingezogen wird, geformt. Nachteilig an dieser bekannten Blindniete ist deren beschränkte Anwendung auf weiche Materialien und die Notwendigkeit hoher Schliesskräfte zur Bildung des Schliesskopfes an der Blindniete.

Hier will die Erfindung Abhilfe schaffen. Der Erfindung liegt die Aufgabe zugrunde, ein medizinisch anwendbares Fixationsmittel, insbesondere eine chirurgisch anwendbare Blindniete zu schaffen, welche Materialien mit höherer Festigkeit, beispielsweise Titan zulässt und mittels in der Chirurgie vertretbarer Schliesskräfte fixierbar ist.

Die Erfindung löst die gestellte Aufgabe mit einer Vorrichtung zur Frakturbehandlung und/oder zur Fixierung von chirurgischen Implantaten, chirurgischen Fäden oder Geweben im oder am Knochen, welche die Merkmale des Anspruchs 1 aufweist.

Eine bevorzugte Ausführungsform der erfindungsgemässen Vorrichtung umfasst im wesentlichen
A) ein Schliesselement mit einem Schaft und endständig einem fest mit dem Schaft verbundenen oder beispielsweise mittels einer Gewindeverbindung mit dem Schaft verbindbaren Kopf; und
B) eine Niete, welche koaxial zu einer Zentralachse einen Nietenschaft und eine die Niete koaxial durchdringende Durchgangsöffnung umfasst.

Der Schaft des Schliesselementes ist koaxial in der Durchgangsöffnung verschiebbar so dass der Kopf am vorderen Schaftende axial zur Anlage bringbar ist. Kopf und Schaft des Schliesselementes können zweiteilig oder einteilig ausgebildet sein. Ferner umfasst der Nietenschaft aussen mindestens zwei Nuten, welche sich vom vorderen Schaftende her parallel zur Zentralachse über eine Länge L in Richtung des hinteren Endes der Niete erstrecken. Die Nuten dienen als Sollbruchstellen, so dass bei weiterer Verschiebung des Schliesselementes in Richtung des Nietenkopfes der Nietenschaft auf einem Teil seiner Gesamtlänge durch den Kopf des Schliesselementes in Verankerungszungen aufgeteilt wird. Das Verhältnis der Länge L zur Gesamtlänge des Nietenschaftes beträgt zwischen 20% und 90%, so dass sich die Verankerungszungen radial auf eine Fläche F ausdehnen können, welche das 3-fache bis 20-fache der Querschnittsfläche des Nietenschaftes betragen kann.

Bei der erfindungsgemässen Vorrichtung umfasst der Nietenschaft Schlitze, welche einerseits am vorderen Schaftende in die Durchgangsöffnung und andererseits parallel zur Zentralachse in die Nuten münden. Durch diese Schlitze wird das Aufreissen des Nietenschaftes begünstigt.

In einer anderen Ausführungsform der erfindungsgemässen Vorrichtung umfasst die Niete am hinteren Ende einen Nietenkopf, welcher fest mit der Niete verbunden sein kann oder beispielsweise mittels einer Gewindeverbindung mit dem Schaft verbindbar sein kann.

In einer weiteren Ausführungsform der erfindungsgemässen Vorrichtung liegen die Werkstoffkennwerte des Nietenmaterials innerhalb von Eckdaten, welche einen Bereich des Verhältnisses Zugfestigkeit (Rₘ in N/mm²) zu Bruchdehnung (A5 in %) von 10:1 bis 50 : 1, vorzugsweise 10 : 1 bis 30 : 1 einschliessen.

In wiederum einer weiteren Ausführungsform der erfindungsgemässen Vorrichtung werden die geometrischen Abmessungen der Niete so gewählt, dass das Verhältnis des Aussendurchmessers dₐ des Nietenschaftes zum Durchmesser d der Durchgangsöffnung in einem Bereich von 1,1 : 1 bis 2,5 : 1, vorzugsweise von 1,5 : 1 bis 2 : 1 liegt.

Das Verhältnis der radialen Tiefe t der Nuten zur Wandstärke des Nietenschaftes liegt erfindungsgemäß in einem Bereich von 1 : 1,2 bis 1 : 2,5, vorzugsweise von 1 : 1,7 bis 1 : 2,3. Die Wandstärke lässt sich aus der Differenz des Aussendurchmessers dₐ und dem Durchmesser d ermitteln.

Nieten mit diesen geometrischen Verhältnissen haben dann geometrische Abmessungen, welche innerhalb der folgenden Eckdaten liegen:
Aussendurchmesser dₐ des Nietenschaftes : 2 bis 12 mm, vorzugsweise 3 bis 8 mm;
Durchmesser d der Durchgangsöffnung: 1 bis 8 mm, vorzugsweise 1,5 bis 5 mm;
Wandstärke des Nietenschaftes: 0,2 bis 4 mm, vorzugsweise 0,5 bis 2 mm; und
Radiale Tiefe t der Nuten: 0,1 bis 3 mm, vorzugsweise 0,2 bis 1 mm.

In einer weiteren Ausführungsform der erfindungsgemässen Vorrichtung haben die Nuten senkrecht zur Zentralachse einen dreieckförmigen Querschnitt, wobei der Spitzenwinkel des Dreieckes im Nutengrund innerhalb eines Bereiches von 30° bis 80°, vorzugsweise von 40° bis 70° liegt. Die Anzahl der gleichmässig auf dem Umfang des Nietenschaftes verteilten Nuten liegt sinnvollerweise in einem Bereich von 3 bis 8, vorzugsweise 3 bis 5. Aus der Anzahl der Nuten ergibt sich bei der fixierten Niete auch die Anzahl der Verankerungszungen.

Das Nietenmaterial ist vorzugsweise metallisch und umfasst folgende Materialien respektive Legierungen:
a) Materialien auf Eisenbasis, vorzugsweise Stahl;
b) Materialien auf Titanbasis, vorzugsweise Ti c.p. und Titanlegierungen;
c) Materialien auf Kobaltbasis, vorzugsweise Kobaltlegierungen;
d) Materialien auf Tantalbasis, vorzugsweise Tantallegierungen; und
e) Materialien auf Zirkonbasis, vorzugsweise Zirkonlegierungen.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung:
- eine homogenere Krafteinleitung in den Knochen als bei Schrauben möglich ist; und
- eine stabilere Verankerung als bei Knochenschrauben auch bei schlechter Knochenqualität herstellbar ist.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Ansicht der erfindungsgemässen Vorrichtung;
Fig. 2 einen Längsschnitt durch die in Fig. 1 dargestellte Ausführungsform der erfindungsgemässen Vorrichtung;
Fig. 3 den Schnitt I-I durch die in den Fig. 1 und 2 dargestellte Ausführungsform der erfindungsgemässen Vorrichtung;
Fig. 4 eine perspektivische Darstellung durch die in den Fig. 1 bis 3 dargestellte Ausführungsform der erfindungsgemässen Vorrichtung; und
Fig. 5 einen Längsschnitt durch eine andere Ausführungsform der erfindungsgemässen Vorrichtung.

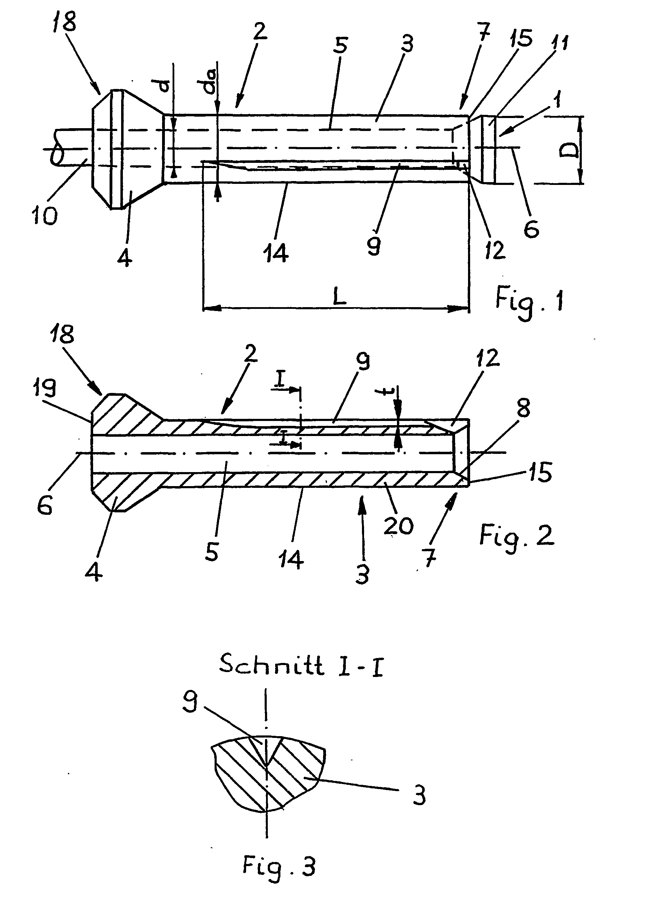

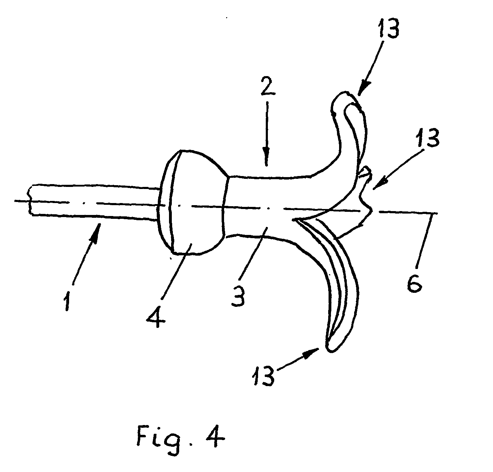

In Fig. 1 sind eine Niete 2 und ein Schliesselement 1 gemäss einer Ausführungsform der erfindungsgemässen Vorrichtung dargestellt. Die Niete 2 weist eine Zentralachse 6 auf und besteht aus einem parallel zur Zentralachse 6 verlaufenden zylindrischen Nietenschaft 3, der nicht zwangsläufig kreiszylindrisch sein muss, einem fest mit dem Nietenschaft 3 verbundenen Nietenkopf 4 und einer entlang der Zentralachse 6 koaxial die Niete 2 durchdringenden zylindrischen Durchgangsöffnung 5. Der Nietenschaft 3 weist einen Aussendurchmesser dₐ auf und umfasst Nuten 9, welche parallel zur Zentralachse 6 verlaufen, sich vom vorderen Schaftende 7 über eine Länge L erstrecken und von der äusseren Mantelfläche 14 eine Tiefe t aufweisen. Die Tiefe t ist so bemessen, dass die durch die Durchmesser dₐ und d und die Tiefe t definierte Wandstärke beim Schliessen der Niete 2 mit dem Schliesselement 1 eine Trennung der Niete 2 auf dem zu schliessenden Teil in separate Verankerungszungen 13 (Fig. 4) zulässt, wobei die Anzahl der Verankerungszungen 13 der Zahl der auf der äusseren Mantelfläche 14 der Niete 2 angebrachten Nuten 9 entspricht. Bei der hier dargestellten Ausführungsform der Niete 2 entspricht die Wandstärke des Nietenschaftes 3 14% des Aussendurchmessers dₐ. Von der vorderen Stirnfläche 15 des Nietenschaftes 3 dringen parallel zur Zentralachse 6 Schlitze 12, welche einerseits am vorderen Schaftende 7 in die Durchgangsöffnung 5 und andererseits parallel zur Zentralachse 6 in die Nuten 9 münden, in den Nietenschaft 3 ein. Das Schliesselement 1 umfasst einen zur Zentralachse 6 parallelen Schaft 10 und endständig einen Kopf 11, welcher kegelstumpfförmig in den Schaft 10 mündet. Das Schliessen der Niete 2 erfolgt nach dem Einführen der Niete 2, beispielsweise in eine Knochenplatte und einen Knochen (nicht gezeichnet) mittels des Schliesselementes 1. Der Kopf 11 des Schliesselementes 1 weist einen Durchmesser D auf, welcher grösser als der Durchmesser d der Durchgangsöffnung 5 ist, so dass der Kopf 11 des Schliesselementes 1 mittels einer auf den Schaft 10 ausgeübten Zugkraft in den Innenkonus 8 (Fig. 2) der Durchgangsöffnung 5 eingepresst wird. Beim Einpressen des weiter werdenden kegelstumpfförmigen Kopfes 11 des Schliesselementes 1 in den Innenkonus 8 (Fig. 2) in der Durchgangsöffnung 5 wird die Wand 20 (Fig. 2) des Nietenschaftes 3 aufgeweitet und bei weiterem Einpressen des Kopfes 11 des Schliesselementes 1 an den durch die Nuten 9 gebildeten Sollbruchstellen in die Verankerungszungen 13 (Fig. 4) aufgetrennt.

In Fig. 2 ist die zur oben beschriebenen Ausführungsform der erfindungsgemässen Vorrichtung gehörende Niete 2 dargestellt. Die Niete 2 umfasst eine Zentralachse 6, einen kreiszylindrischen Nietenschaft 3 mit einem die Zentralachse 6 schneidenden, vorderen Schaftende 7 und am hinteren, die Zentralachse 6 schneidenden Ende 18 endständig einen ebenfalls kreiszylindrischen Nietenkopf 4. Am vorderen Schaftende 7 ist eine senkrecht zur Zentralachse 6 stehende vordere Stirnfläche 15. Der Nietenkopf 4 mündet gegen das vordere Schaftende 7 gerichtet sich konvex oder kegelstumpfförmig verjüngend in den Nietenschaft 3 und ebenfalls sich konvex oder kegelstumpfförmig verjüngend in die hintere, endständig am hinteren Ende 18 liegende Stirnfläche 19 der Niete 2. Koaxial zur Zentralachse 6 und die Niete 2 vom vorderen Schaftende 7 bis zum hinteren Ende 18 durchdringend, ist eine Durchgangsöffnung 5 angebracht. Die Durchgangsöffnung 5 mündet am vorderen Schaftende 7 mit dem Innenkonus 8 sich erweiternd in die vordere Stirnfläche 15. Parallel zur Zentralachse 6 sind Nuten 9 angebracht, welche vom vorderen Schaftende 7 bis zu einer Länge L (Fig. 1) in den Nietenschaft 3 eindringen und senkrecht zur Zentralachse 6 einen V-förmigen Querschnitt (Fig.3) aufweisen. Zudem dringen von der vorderen Stirnfläche 15 her ebenfalls parallel zur Zentralachse 6 Schlitze 12 in den Nietenschaft 3 ein. Diese Schlitze 12 weisen senkrecht zur Zentralachse 6 einen rechteckigen Querschnitt (Fig. 1) auf und sind parallel zur Zentralachse 6 kreissegmentförmig ausgestaltet. Die Schlitze 12 verlaufen parallel zur Zentralachse 6 und münden in die vordere Stirnfläche 15, in die äussere Mantelfläche 14 und in die Nuten 9.

Fig. 4 zeigt die Niete 2 mit Zentralachse 6, Nietenkopf 4, aufgeweitetem Nietenschaft 3 und Schliesselement 1 in geschlossenem Zustand der Niete 2. Der Nietenschaft 3 ist auf der Länge L (Fig. 1) aufgeweitet und umfasst drei Verankerungszungen 13.

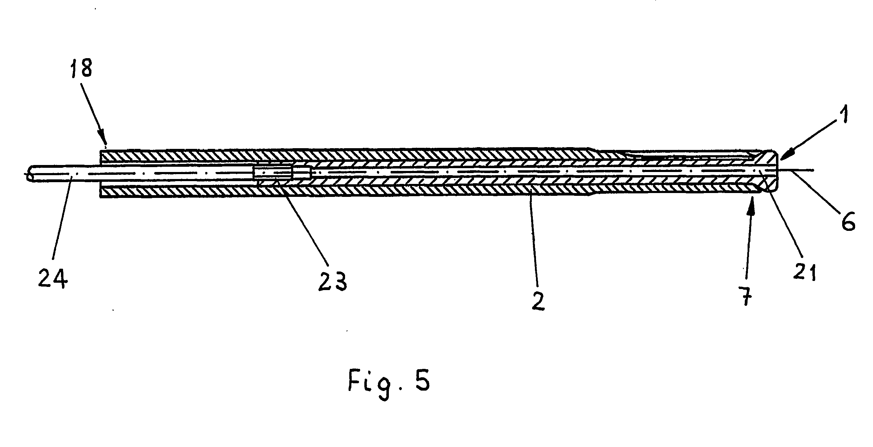

In Fig. 5 ist eine Ausführungsform der Niete 2 dargestellt, welche sich von den in den Fig. 1 und 2 dargestellten Ausführungsformen nur darin unterscheidet, dass die Niete 2 am hinteren Ende 18 keinen Nietenkopf 4 (Fig. 1 und 2) umfasst. Ferner durchdringt das Schliesselement 1 die Niete 2 nur auf einem Teil deren Gesamtlänge und ist mit einer das Schliesselement 1 koaxial durchdringenden Bohrung 21 ausgestattet, welche am dem Kopf 11 axial entgegengesetzt liegenden Ende 22 mit einem koaxialen Innengewinde 23 versehen ist. Ein Kirschnerdraht 24 ist in dieses Innengewinde einschraubbar und lässt sich nach dem Schliessen der Niete 2 wieder aus dem Innengewinde 23 herausschrauben.

## Patentansprüche

1. Vorrichtung zur Frakturbehandlung an Knochen und/oder zur Fixierung von chirurgischen Implantaten, chirurgischen Fäden oder Geweben im oder am Knochen mit einer Niete (2), welche koaxial zu einer Zentralachse (6) einen Nietenschaft (3) mit einer äusseren Mantelfläche (14), ein hinteres, die Zentralachse (6) schneidendes Ende (18), ein dem hinteren Ende (18) axial entgegengesetzt liegendes, vorderes Schaftende (7) und eine die Niete (2) koaxial durchdringende Durchgangsöffnung (5) umfasst, **dadurch gekennzeichnet, dass** der Nietenschaft (3) mindestens zwei Nuten (9) umfasst, welche parallel zur Zentralachse (6) verlaufen, sich vom vorderen Schaftende (7) her auf einer Länge L in Richtung des hinteren Endes (18) erstrecken und von der äusseren Mantelfläche (14) aus gemessen eine radiale Tiefe t aufweisen, wobei
A) der Nietenschaft (3) Schlitze (12) aufweist, welche einerseits am vorderen Schaftende (7) in die Durchgangsöffnung (5) und andererseits parallel zur Zentralachse (6) in die Nuten (9) münden; und
B) das Verhältnis der radialen Tiefe t der Nuten (9) zur Wandstärke des Nietenschaftes (3) in einem Bereich von 1 : 1,2 bis 1 : 2,5 liegt.

2. Vorrichtung nach Anspruch 1, **dadurch gekennzeichnet, dass** das Verhältnis der Länge L zur Gesamtlänge des Nietenschaftes zwischen 20% bis 90% beträgt.

3. Vorrichtung nach Anspruch 1 oder 2, **dadurch gekennzeichnet, dass** das Verhältnis des Aussendurchmessers dₐ des Nietenschaftes (3) zum Durchmesser d der Durchgangsöffnung (5) in einem Bereich von 1,1 : 1 bis 2,5 : 1 liegt.

4. Vorrichtung nach Anspruch 3, **dadurch gekennzeichnet, dass** das Verhältnis des Aussendurchmessers dₐ des Nietenschaftes (3) zum Durchmesser d der Durchgangsöffnung (5) in einem Bereich von 1,5 : 1 bis 2 : 1 liegt.

5. Vorrichtung nach einem der Ansprüche 1 bis 4, **dadurch gekennzeichnet, dass** das Verhältnis der radialen Tiefe t der Nuten (9) zur Wandstärke des Nietenschaftes (3) in einem Bereich von 1 : 1,7 bis 1 : 2,3 liegt.

6. Vorrichtung nach einem der Ansprüche 1 bis 5, **dadurch gekennzeichnet, dass** sie ein Schliesselement (1) umfasst, welches einen Schaft (10) und einen endständigen Kopf (11) aufweist, so dass der Schaft (10) des Schliesselementes (1) koaxial in der Durchgangsöffnung (5) verschiebbar ist und der Kopf (11) am vorderen Schaftende (7) axial zur Anlage bringbar ist.

7. Vorrichtung nach einem der Ansprüche 1 bis 6, **dadurch gekennzeichnet, dass** die Niete (2) am hinteren Ende (18) einen Nietenkopf (4) umfasst.

## Claims

1. A device for fracture treatment on bones and/or for fixing surgical implants, surgical sutures or tissues in or on the bone including a rivet (2) which comprises, extending coaxially to a central axis (6), a rivet shank (3) having an outer lateral surface (14), a rear end portion (18) intersecting the central axis (6), a shank front end portion (7) axially opposite to the rear end portion (18), and a through hole (5) coaxially penetrating the rivet (2),
**characterized in that** the rivet shank (3) comprises at least two grooves (9) running parallel to the central axis (6) which extend from the shank front end portion (7) over a length L in the direction of the rear end portion (18) and which have a radial depth t, measured from the outer lateral surface (14), whereby
A) the rivet shank (3) comprises slits (12) which on the shaft front end portion (7) lead to the through hole (5), whereas on the other hand they lead parallel to the central axis (6) to the grooves (9); and
B) the ratio between the radial depth t of the grooves (9) and the wall thickness of the rivet shank (3) is in a range of between 1 : 1.2 and 1 : 2.5.

2. A device as claimed in claim 1, **characterised in that** the ratio between the length L and the total length of the rivet shank is comprised between 20 and 90 percent.

3. A device as claimed in claim 1 or 2, **characterised in that** the ratio between the outside diameter dₐ of the rivet shank (3) and the diameter d of the through hole (5) is in a range of between 1.1 : 1 and 2.5 : 1.

4. A device as claimed in claim 3, **characterised in that** the ratio between the outside diameter dₐ of the rivet shank (3) and the diameter d of the through hole (5) is in a range of between 1.5:1 and 2:1.

5. A device as claimed in any of the claims 1 to 4, **characterised in that** the ratio between the radial depth t of the grooves (9) and the wall thickness of the rivet shank (3) is in a range of between 1:1.7 and 1 : 2.3.

6. A device as claimed in any of the claims 1 to 5, **characterised in that** it comprises a closing element (1) having a shank (10) and a head (11) formed in the end portion thereof, so that the shank (10) of the closing element (1) is coaxially displaceable within the through hole (5) and that the head (11) may be axially brought to rest on the shaft front end portion (7).

7. A device as claimed in any of the claims 1 to 6, **characterised in that** the rivet (2) comprises a rivet head (4) formed on the rear end portion (18) thereof.

## Revendications

1. Dispositif de traitement des fractures osseuses et/ou de fixation d'implants chirurgicaux, de fils ou de tissus chirurgicaux dans ou sur l'os, avec un rivet (2) qui, de façon coaxiale par rapport à un axe central (6), comprend une tige de rivet (3) comportant une surface latérale extérieure (14), comprend une extrémité arrière (18) coupant l'axe central (6), une extrémité avant (7) de la tige, placée à l'opposé, axialement, de l'extrémité arrière (18), et une ouverture de passage (5) traversant coaxialement le rivet (2),
**caractérisé en ce que** la tige de rivet (3) comprend au moins deux gorges (9) qui passent parallèlement à l'axe central (6), s'étendent, à partir de l'extrémité avant (7) de la tige, sur une longueur L, en direction de l'extrémité arrière (18), et présentent une profondeur radiale t mesurée à partir de la surface latérale extérieure (14), où
A/ la tige de rivet (3) comporte des fentes (12) qui débouchent, d'une part, dans l'ouverture de passage (5) au niveau de l'extrémité avant (7) de la tige et, d'autre part, dans les gorges (9) parallèlement à l'axe central (6); et
B/ le rapport de la profondeur radiale t des gorges (9) relativement à l'épaisseur de paroi de la tige de rivet (3) se situe dans une plage de 1:1,2 à 1:2,5.

2. Dispositif selon la revendication 1, **caractérisé en ce que** le rapport de la longueur L relativement à la longueur totale de la tige de rivet est compris entre 20% et 90%.

3. Dispositif selon la revendication 1 ou 2, **caractérisé en ce que** le rapport du diamètre extérieur dₐ de la tige de rivet (3) relativement au diamètre d de l'ouverture de passage (5) se situe dans une plage de 1,1:1 à 2,5:1.

4. Dispositif selon la revendication 3, **caractérisé en ce que** le rapport du diamètre extérieur dₐ de la tige de rivet (3) relativement au diamètre d de l'ouverture de passage (5) se situe dans une plage de 1,5:1 à 2:1.

5. Dispositif selon l'une des revendications 1 à 4, **caractérisé en ce que** le rapport de la profondeur radiale t des gorges (9) relativement à l'épaisseur de paroi de la tige de rivet (3) se situe dans une plage de 1:1,7 à 1:2,3.

6. Dispositif selon l'une des revendications 1 à 5, **caractérisé en ce qu'**il comprend un élément de fermeture (1) qui comporte une tige (10) et une tête terminale (11), de sorte que la tige (10) de l'élément de fermeture (1) peut être déplacée de façon coaxiale dans l'ouverture de passage (5), et la tête (11) peut être placée en appui, axialement, sur l'extrémité avant (7) de la tige.

7. Dispositif selon l'une des revendications 1 à 6, **caractérisé en ce que** le rivet (2) comprend une tête de rivet (4) au niveau de l'extrémité arrière (18).
